## Europäisches Patentamt
## European Patent Office
## Office européen des brevets

(11) Veröffentlichungsnummer: **0 445 244 B1**

(12) **EUROPÄISCHE PATENTSCHRIFT**

(45) Veröffentlichungstag der Patentschrift: **20.09.95**

(51) Int. Cl.⁶: **C07J 1/00**

(21) Anmeldenummer: **90913267.2**

(22) Anmeldetag: **06.09.90**

(86) Internationale Anmeldenummer:
**PCT/DE90/00690**

(87) Internationale Veröffentlichungsnummer:
**WO 91/04264 (04.04.91 91/08)**

(54) **VERFAHREN ZUR HERSTELLUNG VON 3-HYDROXY-1,3,5(10)-ESTRATRIEN-17-ON.**

(30) Priorität: **20.09.89 DE 3931820**

(43) Veröffentlichungstag der Anmeldung:
**11.09.91 Patentblatt 91/37**

(45) Bekanntmachung des Hinweises auf die
Patenterteilung:
**20.09.95 Patentblatt 95/38**

(84) Benannte Vertragsstaaten:
**AT BE CH DE DK ES FR GB IT LI LU NL SE**

(56) Entgegenhaltungen:
**DE-A- 2 537 254**

(73) Patentinhaber: **SCHERING AKTIENGESELL-
SCHAFT
Müllerstrasse 170/178
D-13353 Berlin (DE)**

(72) Erfinder: **BUBACK, Michael
Alte Dorfstr. 57e
D-3406 Bovenden (DE)**
Erfinder: **HADER, Josef
Hattenheimer Str. 14
D-1000 Berlin 28 (DE)**
Erfinder: **VÖGELE, Hans-Peter
Am Kalten Born 28
D-3400 Göttingen (DE)**
Erfinder: **AL-MASSRI, Zakarya
Albrecht Thaer Weg 22 b
D-3400 Göttingen (DE)**

Rank Xerox (UK) Business Services
(3.10/3.09/3.3.3)

**Beschreibung**

Die Erfindung betrifft ein Verfahren zur Herstellung von 3-Hydroxy-1,3,-5(10)-estratrien-17-on (= Estron) sowie eine Vorrichtung zur Durchführung dieses Verfahrens.

Aus der deutschen Patentschrift 25 37 254 ist ein Verfahren zur Herstellung von 3-Hydroxy-1,3,5-(10)-estratrien-17-on durch Pyrolyse von 1,4-Androstadien-3,17-dion in Gegenwart von Kohlenwasserstoffen als Wasserstoff-Donator vorbekannt, dessen Merkmale sind, daß man einen auf eine Temperatur von 450-850° C vorgeheizten Kohlenwasserstoff in einer Mischzone mit einer auf eine Temperatur von bis zu 300° C erhitzten Lösung von 1,4-Androstadien-3,17-dion in einem Kohlenwasserstoff mischt, das Reaktionsgas in einer Pyrolysezone bei einer Verweilzeit von 0,001 bis 60 Sekunden auf Temperaturen von 450-700° C erhitzt und das aus der Pyrolysezone austretende Produkt abkühlt. In den Ausführungsbeispielen dieser Patentschrift wird erwähnt, daß man als Kohlenwasserstoff auch 1,2,3,4-Tetrahydronaphthalin verwenden kann, und daß man die Reaktion unter einem Druck von bis zu $3 \times 10^6$ Pa durchführen kann.

Der wesentlichste Nachteil dieses vorbekannten Verfahrens ist darin zu sehen, daß die Pyrolysezone bei der Durchführung des Verfahrens durch Teer- und Rußbildung rasch verstopft und die häufig erforderliche Reinigung dieser Zone eine unter ökonomischen Bedingungen tragbare Durchführung des Verfahrens erschwert. Hinzu kommt, daß man bei den vorbekannten Verfahren, bei denen die Reaktion in der Gasphase durchgeführt wird, nur eine relativ geringe Raum-Zeit-Ausbeute an Verfahrensprodukt erhält.

Es wurde nun gefunden, daß man überraschenderweise diesen Nachteil des vorbekannten Verfahrens vermeiden kann, wenn man die Pyrolyse unter einem Druck von $3,5 \times 10^6$ - $3 \times 10^8$ Pa durchführt.

Unter diesen erfindungsgemäßen Bedingungen des Verfahrens erzielt man darüber hinaus überraschend hohe Ausbeuten an Verfahrensprodukt, vermeidet Uberhitzungen und erhält eine hohe Raum-Zeit-Ausbeute an Verfahrensprodukt.

Das erfindungsgemäße Verfahren wird unter Bedingungen durchgeführt, bei denen sowohl die kritische Temperatur des 1,2,3,4-Tetrahydronaphthalins von 446° C als auch dessen kritischer Druck von etwa $3,5 \times 10^6$ Pa überschritten ist, so daß die Reaktion in einer überkritischen fluiden Phase durchgeführt wird.

Das erfindungsgemäße Verfahren kann in den Vorrichtungen durchgeführt werden, die in der deutschen Patentschrift 25 37 254 beschrieben sind, vorausgesetzt, daß diese geeignete Abmessungen besitzen und aus einem Material gefertigt sind, welches einem Druck von $3,5 \times 10^6$ Pa bis 3

$\times 10^8$ Pa standhält und daß es zwischen dem Wärmeaustauscher und dem Auffanggefäß ein Ventil enthält, welches die Aufrechterhaltung des gewünschten Druckes in der Pyrolysezone gewährleistet.

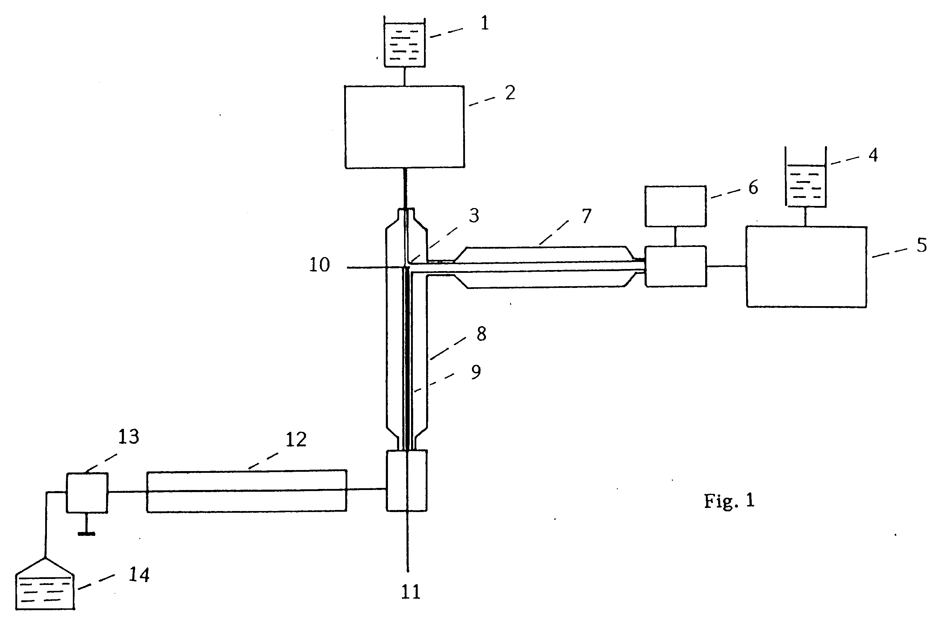

Eine sehr geeignete, robuste und einfache Vorrichtung ist in der beigefügten Figur 1 dargestellt. Sie ist im wesentlichen aus rostfreiem Stahl gefertigt.

Aus einem Vorratsgefäß 1, welches die Lösung von 1,4-Androstadien-3,17-dion in 1,2,3,4-Tetrahydronaphthalin enthält, wird diese über eine Pumpe 2 in die Mischzone 3 eingespeist. Gleichzeitig wird aus einem zweiten Vorratsgefäß 4 über eine Pumpe 5 1,2,3,4-Tetrahydronaphthalin durch eine mit einem Druckmeßgerät versehene Vorheizzelle 7 geleitet und nach Erreichung der gewünschten Temperatur ebenfalls in die Mischzone 3 eingespeist. Die Mischzone 3 selbst befindet sich im Kopf des elektrisch beheizbaren Pyrolysereaktors 8, welcher einen 130 mm langen Ringspalt 9 von 4,5 mm äußerem und 4,2 mm innerem Durchmesser enthält. Am Kopf des Ringspalts und an dessen Ende ist jeweils ein Thermoelement 10 und 11 angeordnet.

Nach Verlassen des Pyrolysereaktors wird die Reaktionsmischung in einer Kühlvorrichtung 12 gekühlt und gelangt über ein Ventil 13, welches die Aufrechterhaltung des gewünschten Druckes in der Pyrolysezone gewährleistet, in den Auffangbehälter 14.

Es ist für den Fachmann selbstverständlich, daß diese Vorrichtung den Bedürfnissen entsprechend modifiziert werden kann. So kann man die Vorrichtung beispielsweise mit einer zusätzlichen Vorheizzelle versehen, die es erlaubt, auch die Lösung von 1,4-Androstadien-3,17-dion in 1,2,3,4-Tetrahydronaphthalin zu erhitzen, ehe sie in Mischzone 3 eingespeist wird. Andererseits ist es aber auch beispielsweise möglich, den Ringspaltdurchmesser und/oder die Ringspaltlänge zu verändern. Eine Vergrößerung des Ringspaltdurchmessers hat den Vorzug, daß die Durchflußrate an Reaktionsmischung wesentlich erhöht wird.

Die nach dem erfindungsgemäßen Verfahren erzielbaren Ausbeuten sind entscheidend abhängig von der Reaktionstemperatur in der Pyrolysezone (450° C -700° C; vorzugsweise 540° C bis 650° C) und der Verweilzeit der Reaktionsmischung in der Pyrolysezone. Diese beträgt in der Regel nur etwa 0,1 Sekunden bis etwa 3 Sekunden. Eine zu lange Verweilzeit ist nachteilig, da das gebildete Estron pyrolytisch gespalten wird. Die genaue Einhaltung der optimalen Reaktionszeit in der Pyrolysezone ist entscheidend davon abhängig, wie rasch die gewünschte Reaktionstemperatur des umzusetzenden 1,4-Androstadien-3,17-dions erreicht werden kann. Diese kann offenkundig um so rascher erreicht werden, je größer das Verhältnis des vorerhitzten 1,2,3,4-Tetrahydronaphthalins zur 1,4-Androstadien-3,17-dion-Lösung ist. Je größer dieses Verhältnis ist, desto ungünstiger wird andererseits die ökonomische Durchführbarkeit des Verfahrens, da die Raum-Zeit-Ausbeute an Verfahrensprodukt entsprechend abnimmt. Es hat sich nach eigenen Versuchen als zweckmäßig erwiesen, wenn man pro Volumeneinheit 1,4-Androstadien-3,17-dion-Lösung 2 bis 10 Volumeneinheiten vorzuerhitzendes 1,2,3,4-Tetrahydronaphthalin verwendet. Es ist für den Fachmann offenkundig, daß die gewünschte Reaktionstemperatur in der Pyrolysezone um so rascher erreicht wird, je konzentrierter die 1,4-Androstadien-3,17-dion-Lösung ist und je stärker diese vorerhitzt wurde. Bei 1,4-Androstadien-3,17-dion-Lösungen, die nicht vorgewärmt werden, ist es nach eigenen Versuchen zweckmäßig, solche zu verwenden, die etwa 20 bis 100 g 1,4-Androstadien-3,17-dion pro 1000 ml 1,2,3,4-Tetrahydronaphthalin enthalten. Eigene Versuche mit vorerhitzter 1,4-Androstadien-3,17-dion-Lösung wurden bislang noch nicht durchgeführt.

Zur Erzielung optimaler Ausbeuten ist es ebenfalls wichtig, daß die Reaktionsmischung nach erfolgter Umsetzung abgekühlt wird.

Erfindungsgemäß wird die Pyrolyse unter einem Druck von $3,5 \times 10^6$ bis $3 \times 10^8$ Pa und insbesondere unter einem Druck von $5 \times 10^6$ bis $2 \times 10^7$ Pa durchgeführt. Die Anwendung noch höherer Drucke scheint keine Vorteile zu bringen, sondern es könnte im Gegenteil so sein, daß durch die Anwendung sehr hohen Druckes die Bildung von Nebenprodukten etwas erhöht wird. Hinzu kommt, daß die technische Durchführung des Verfahrens apparativ um so aufwendiger wird, je höher der Druck ist, bei dem das Verfahren durchgeführt wird.

Das nachfolgende Ausführungsbeispiel dient zur näheren Erläuterung der Erfindung.

Beispiel

Zur Durchführung des Verfahrens wird die bereits beschriebene Vorrichtung verwendet.

Aus dem Vorratsgefäß (4) werden mittels der Pumpe (5) 30 ml 1,2,3,4-Tetrahydronaphthalin pro Minute in die Vorheizzelle (7) eingeleitet, wo sie auf eine Temperatur erhitzt werden, die etwa 50° C über der letztlich gewünschten Reaktionstemperatur liegt und dann in die Mischzone (3) des auf die gewünschte Reaktionstemperatur erhitzten Pyrolysereaktors (8) eingespeist. Gleichzeitig werden aus dem Vorratsgefäß (1), welches eine Lösung von 50 g 1,4-Androstadien-3,17-dion in 1000 ml 1,2,3,4-Tetrahydronaphthalin enthält, mittels der Pumpe (2) 10 ml Lösung pro Minute in die Mischzone (3) des Pyrolysereaktors 8 eingespeist.

Nach Durchströmen des Ringspaltes (9) wird die Reaktionsmischung in der Kühlvorrichtung (12) gekühlt und gelangt über das Ventil (13), welches zur Einstellung des gewünschten Reaktionsdruckes dient, in den Auffangbehälter (14).

Proben der Reaktionsmischung werden mittels gaschromatographischer Analyse auf ihren 1,4-Androstadien-3,17-dion- und Estrongehalt untersucht.

Aus Messungen bei unterschiedlichen Temperaturen und unterschiedlichen Drucken werden folgende drei Beziehungene ermittelt:

1. Für den Geschwindigkeitskoeffizienten erster Ordnung des ADD-Zerfalls die Beziehung

$$\ln (k_{ADD} s^{-1}) = 37,5 - 29762/T$$

(ADD = 1,4-Androstadien-3,17-dion)

2. Für die Selektivität des ADD-Zerfalls zu Estron die Beziehung

$$\ln (\% \ OES/\% \ NPR) = 22.5 - 17\,391/T$$

(OES = Estron; NPR = Nebenprodukte)

3. Für den Geschwindigkeitskoeffizienten erster Ordnung des Zerfalls von Estron die Beziehung:

$$\ln(k_{oes} s^{-1}) = 21,3 - 19820/T$$

(Die Temperaturen in den vorausgehenden Formeln sind jeweils in Kelvin einzusetzen).

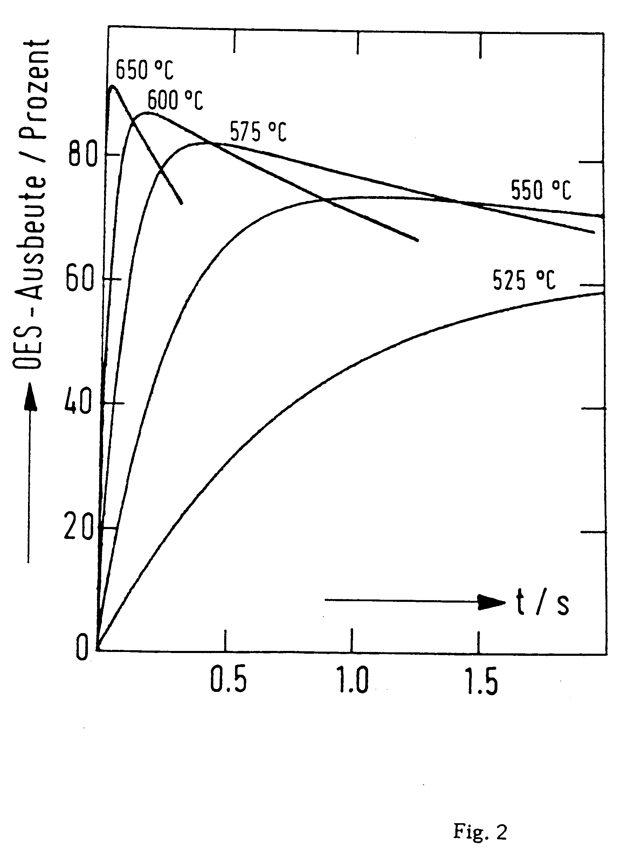

Aus diesen Beziehungen ergeben sich die in Figur 2 dargestellten Ausbeuten an Estron (OES)in Abhängigkeit von der Reaktionszeit und Reaktionstemperatur.

**Patentansprüche**

1. Verfahren zur Herstellung von 3-Hydroxy-1,3,5-(10)-estratrien-17-on durch Pyrolyse von 1,4-Androstadien-3,17-dion in Gegenwart von 1,2,3,4-Tetrahydronaphthalin als Wasserstoff-Donator, indem man auf eine Temperatur von 450-850° C vorgeheiztes 1,2,3,4-Tetrahydronaphthalin in einer Mischzone mit einer auf eine Temperatur von bis zu 300° C erhitzten Lösung von 1,4-Androstadien-3,17-dion in 1,2,3,4-Tetrahydronaphthalin mischt, das Gemisch in einer Pyrolysezone bei einer Verweilzeit von 0,001 bis 60 Sekunden auf Temperaturen von 450-700° C erhitzt und das aus der Pyrolysezone aus tretende Produkt abkühlt, dadurch gekennzeichnet, daß man die Pyrolyse unter einem Druck von $3,5 \times 10^6$ bis $3 \times 10^8$ Pa durchführt.

## Claims

1. Process for the manufacture of 3-hydroxy-1,3,5(10)-oestratrien-17-one by pyrolysis of 1,4-androstadiene-3,17-dione in the presence of 1,2,3,4-tetrahydronaphthalene as hydrogen donor, in which process 1,2,3,4-tetrahydronaphthalene pre-heated to a temperature of 450-850°C is mixed in a mixing zone with a solution of 1,4-androstadiene-3,17-dione in 1,2,3,4-tetrahydronaphthalene heated to a temperature of up to 300°C, the mixture is heated in a pyrolysis zone to temperatures of 450-700°C during a residence time of from 0.001 to 60 seconds, and the product leaving the pyrolysis zone is cooled, characterised in that the pyrolysis is carried out under a pressure of from $3.5 \times 10^6$ to $3 \times 10^8$ Pa.

## Revendications

1. Procédé de préparation de la 3-hydroxy-1,3,5-(10)-estratriène-17-one par pyrolyse de la 1,4-androstadiène-3,17-dione en présence de 1,2,3,4-tétrahydronaphtalène comme donneur d'hydrogène, dans lequel on mélange dans une zone de mélange du 1,2,3,4-tétrahydronaphtalène préalablement porté à 450-850°C avec une solution de 1,4-androstadiène-3,17-dione dans du 1,2,3,4-tétrahydronaphtalène portée à une température s'élevant jusqu'à 300°C, puis on chauffe le mélange à des températures de 450-700°C dans une zone de pyrolyse pendant des durées de séjour de 0,001 à 60 secondes et on refroidit le produit sortant de la zone de pyrolyse, procédé caractérisé en ce que l'on effectue la pyrolyse sous une pression de $3,5 \times 10^6$ à $3 \times 10^8$ Pa.

Fig. 1

Fig. 2